# EUROPÄISCHE PATENTSCHRIFT

(11) **EP 3 119 747 B1**
(45) Veröffentlichungstag und Bekanntmachung des Hinweises auf die Patenterteilung: **29.08.2018**
(21) Anmeldenummer: 15710775.6
(22) Anmeldetag: 18.03.2015
(51) Int. Cl.: C07D 213/61

(54) **VERFAHREN ZUR HERSTELLUNG VON N-[(6-CHLORPYRIDIN-3-YL)METHYL]-2,2-DIFLUORETHAN-1-AMIN DURCH ALKYLIERUNG VON 2,2-DIFLUORETHYLAMIN**
METHOD FOR THE PREPARATION OF N- [(6-CHLOROPYRIDIN-3-YL) METHYL] -2,2-DIFLUORETHAN-1-AMINE BY ALKYLATION OF 2,2-DIFLUORETHYLAMINE
PROCÉDÉ DE FABRICATION DE N-[(6-CHLORPYRIDIN-3-YL)MÉTHYL]-2,2-DIFLUORÉTHAN-1-AMINE PAR ALKYLATION DE 2,2-DIFLUORÉTHYLAMINE

(30) Priorität: 21.03.2014 EP 14161110
(43) Veröffentlichungstag der Anmeldung: 25.01.2017
(73) Patentinhaber: Bayer CropScience Aktiengesellschaft, 40789 Monheim am Rhein (DE)
(72) Erfinder: MORADI, Wahed Ahmed, 40789 Monheim (DE); SCHLEGEL, Günter, 51381 Leverkusen (DE); SCHNATTERER, Albert, 51373 Leverkusen (DE)
(74) Vertreter: BIP Patents
(86) Internationale Anmeldenummer: PCT/EP2015/055639
(87) Internationale Veröffentlichungsnummer: WO 2015/140198

(56) Entgegenhaltungen:
- WO-A1-2007/115644
- WO-A1-2011/157650
- WO-A1-2014/001245

## Beschreibung

Die vorliegende Erfindung betrifft ein neues und ökonomisch wie ökologisch effizientes Verfahren zur Herstellung von N-[(6-Chlorpyridin-3-yl)methyl]-2,2-difluorethan-1-amin ausgehend von 2,2-Difluorethylamin und 2-Chlor-5-(chlormethyl)pyridin (CCMP) in Gegenwart einer anorganischen Base, wobei die anorganische Base NaOH ist. N-[(6-Chlorpyridin-3-yl)methyl]-2,2-difluorethan-1-amin ist eine wichtige Zwischenstufe bei der Herstellung agrochemischer Wirkstoffe (siehe WO-A-2007/115644). Es sind verschiedene Verfahren zur Herstellung von N-[(6-Chlorpyridin-3-yl)methyl]-2,2-difluorethan-1-amin beschrieben. Die bekannten Verfahren haben jedoch, wie nachfolgend beschrieben, diverse Nachteile.

WO-A-2009/036900 offenbart beispielsweise ein Verfahren zur Herstellung von N-[(6-Chlorpyridin-3-yl)methyl]-2,2-difluorethan-1-amin durch Amid-Hydrierung von N-[(6-Chlorpyridin-3-yl)methyl]-2,2-difluoracetamid (Schema 1).

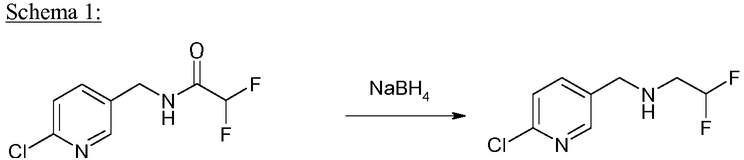

Nachteilig bei diesem Verfahren ist der Einsatz komplexer, sehr teurer Hydride wie Natriumborhydrid, die sicherheitstechnisch sehr aufwendig zu verwenden sind.

WO-A-2009/036901 beschreibt die Reduktion von N-(6-Chlorpyridin-3-yl)methylen-2,2-difluorethanamin durch Wasserstoff (Schema 2).

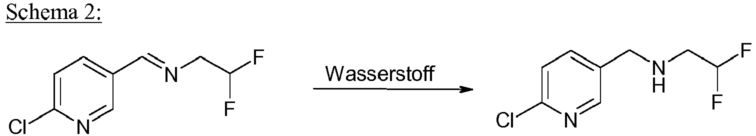

Nachteilig bei diesem Verfahren ist der Einsatz von Wasserstoff, weil auch hier die Verwendung von Wasserstoff sicherheitstechnisch sehr aufwendig ist.

WO-A-2011/157650 beschreibt die Herstellung der 2,2-Difluorethanamin-Derivate ausgehend von 2,2-Difluor-l-halogenethanen mit primären Aminen in Anwesenheit von organischen Basen (Schema 3).

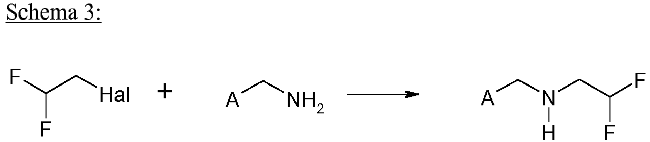

Nachteilig an diesem Verfahren ist, dass die Reaktion in einem Hochdruckapparat durchgeführt werden muss.

Die Patentpublikation WO-A-2007/115644, die sich mit der Herstellung von insektizid wirksamen 4-Aminobut-2-enolidverbindungen befasst, beschreibt die Herstellung von Verbindungen der allgemeinen Formel A-CH₂-NH-R¹, in der A für spezielle Heterocyclen und R¹ für Halogenalkyl steht, durch Alkylierung des Stickstoffs (Schema 4). 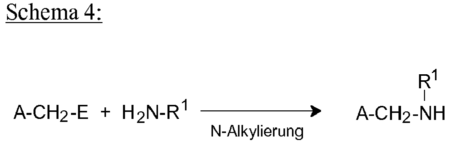 E = Hal, beispielsweise Chlor, Brom, Iod; O-Tosyl, O-Mesyl,

Konkret beschreibt WO-A-2007/115644 die Herstellung von N-[(6-Chlorpyridin-3-yl)methyl]-2,2-difluorethan-l-amin (Verbindung (3)), das ausgehend von CCMP (Verbindung (2)) und 2,2-Difluorethan-l-amin (Verbindung (1)) in Gegenwart von Triethylamin synthetisiert wird (siehe Schema 5). Die Verbindungen (1), (2) und Triethylamin werden dabei in äquimolaren Mengen eingesetzt. Das gewünschte Produkt wird in einer Ausbeute von 53 % erhalten.

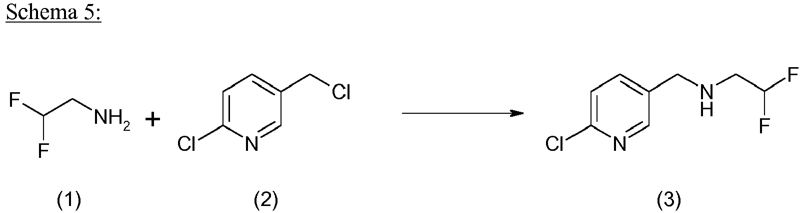

Das in WO-A-2007/116544 beschriebene Verfahren zur Herstellung von Verbindungen der Formel A-CH₂-NH-R¹, in der A für spezielle Heterocyclen und R¹ für Halogenalkyl steht, ist nachteilig, da es während der Umsetzung zu Mehrfachalkylierungen des Stickstoffs kommen kann. Dies führt zu einem Ausbeuteverlust, was auch an der Ausbeute des konkret genannten Beispiels zu erkennen ist. Die Ausbeute betrug lediglich 53 %. Diese Mehrfachalkylierungen können nur durch den Einsatz eines großen Überschusses an Amin reduziert werden. Allerdings ist die destillative Wiedergewinnung des kostspieligen Amins im Regelfall aufwendig und führt zu Verlusten.

WO-A-2014/001245 beschreibt ein Verfahren zur Herstellung von N-[(6-Chlorpyridin-3-yl)methyl]-2,2-difluorethan-l-amin in Gegenwart von N,N-Diisopropylethylamin (Hünigbase). WO-A-2014/001245 offenbart aber nicht, die Reaktion in Gegenwart einer anorganischen Base wie beispielsweise NaOH durchzuführen.

Die Hünigbase ist eine sehr teure, großtechnisch schwerverfügbare Base, die in dem Verfahren gemäß WO-A-2014/001245 in äquimolaren Mengen eingesetzt wird. Die Base liegt nach der Reaktion als Aminhydrochlorid in der wässrigen Phase vor. Zur Wiedergewinnung der freien Base muss die wässrige Phase mit einer anorganischen Base versetzt werden, die Phasen getrennt und die organische Phase destillativ aufbereitet werden. Die destillative Wiedergewinnung der wertvollen Hünigbase ist im Regelfall aufwendig und führt zu Verlusten. Des Weiteren enthält die wässrige Phase ebenfalls die Hünigbase und zwar in Konzentrationen von üblicherweise ca. 1 Gew.-% als freie Hünigbase. Dies führt zu weiteren Verlusten. Darüber hinaus verunreinigt die freie Hünigbase das Abwasser, so dass das Abwasser noch zusätzlich nachbehandelt werden muss.

Wegen der Bedeutung von N-[(6-Chlorpyridin-3-yl)methyl]-2,2-difluorethan-1-amin als Baustein in der Synthese agrochemischer Wirkstoffe ist es jedoch notwendig, ein Verfahren zu finden, das großtechnisch und kostengünstig eingesetzt werden kann. Auch ist es erstrebenswert N-[(6-Chlorpyridin-3-yl)methyl]-2,2-difluorethan-1-amin mit hoher Ausbeute und hoher Reinheit zu erhalten, so dass die Zielverbindung vorzugsweise keiner weiteren - möglicherweise komplexen - Aufreinigung unterzogen werden muss.

Es wurde nun ein Verfahren zur Herstellung von N-[(6-Chlorpyridin-3-yl)methyl]-2,2-difluorethan-1-amin gefunden, das die Nachteile der bekannten Verfahren vermeidet und überdies hinaus noch sehr einfach, kostengünstig und umweltfreundlich durchzuführen ist, so dass es großtechnisch eingesetzt werden kann. Insbesondere kommt das neue erfindungsgemäße Verfahren ohne den Einsatz einer Hünigbase aus.

Die vorliegende Erfindung betrifft somit ein Verfahren zur Herstellung von N-[(6-Chlorpyridin-3-yl)methyl]-2,2-difluorethan-1-amin der Formel (III) 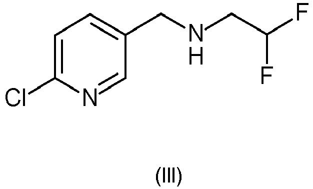 in dem 2,2-Difluorethylamin der Formel (I) 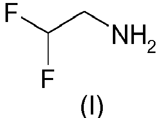 mit 2-Chlor-5-(chlormethyl)pyridin der Formel (II) 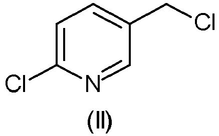 in Gegenwart einer anorganischen Base umgesetzt wird, wobei die anorganische Base NaOH ist

Die erfindungsgemäße Reaktion ist in Schema 6 dargestellt.

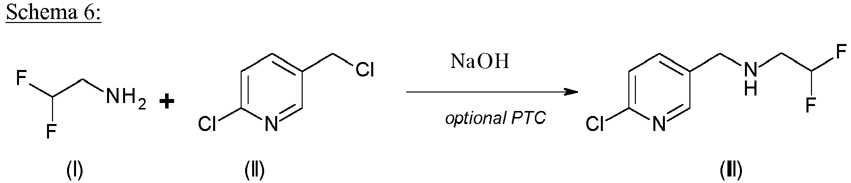

Das gewünschte N-[(6-Chlorpyridin-3-yl)methyl]-2,2-difluorethan-1-amin der Formel (III) wird mit dem erfindungsgemäßen Verfahren mit sehr guten Ausbeuten und in hoher Reinheit erhalten.

Das ist überraschend, da der Fachmann nicht davon ausgehen konnte, dass das eingesetzte 2,2-Difluorethylamin der Formel (I) in Gegenwart von NaOH stabil ist. Vielmehr war zu befürchten, dass die anorganische Base das 2,2-Difluorethylamin der Formel (I) an dem fluorierten Kohlenstoffatom deprotoniert, eine Eliminierungsreaktion bewirkt und das 2,2-Difluorethylaminderivat dann polymerisiert, cyclisiert oder anderweitig abreagiert.

Die gewünschte Verbindung wird dabei in einer Reinheit erhalten, welche eine umfangreiche Aufarbeitung des Reaktionsprodukts im Allgemeinen nicht erforderlich macht. Als Nebenprodukt entsteht das Chlorid des mit der anorganischen Base eingesetzten Alkalimetalls, nämlich NaCl im Falle von NaOH als anorganischer Base. Das ist ein großer Vorteil gegenüber den in WO-A-2014/001245 und WO-A-2007/115644 beschriebenen Verfahren.

Mit dem erfindungsgemäßen Verfahren können zudem mit der kostengünstigen anorganischen Base vergleichbare oder sogar höhere Ausbeute erzielt werden als mit dem in WO-A-2007/115644 oder in WO-A-2014/001245 beschriebenen Verfahren, in denen Triethylamin (WO-A-2007/115644) bzw Hünigbase (WO-A-2014/001245) als tertiäre Stickstoff-Base eingesetzt werden. Dies ist der Fall, wenn NaOH als anorganische Base eingesetzt wird.

Das erfindungsgemäße Verfahren erfolgt in Gegenwart von NaOH.

Bevorzugt wird die anorganische Base in dem erfindungsgemäßen Verfahren in Substanz oder in Form einer wässrigen Lösung eingesetzt. Das Reaktionsgemisch ist daher bevorzugt zweiphasig. Es ist als überraschend anzusehen, dass die anorganische Base trotz der Zweiphasigkeit des Reaktionssystems dennoch ihre Wirksamkeit als Base entfalten kann.

Das Verwenden der anorganischen Base in dem erfindungsgemäßen Verfahren hat den Vorteil, dass so das unverbrauchte 2,2-Difluorethylamin (I) einfach und praktisch vollständig abdestilliert und dem Verfahren als Edukt erneut zugeführt werden kann. Somit kann das Verfahren besonders ressourcenschonend und kostengünstig durchgeführt werden.

Bei Einsatz von NaOH lassen sich höhere Ausbeuten erzielen als mit anderen anorganischen Basen. Dies ergibt sich auch aus den Beispielen. Daher wird NaOH in dem erfindungsgemäßen Verfahren eingesetzt.

Die Umsetzung von 2,2-Difluorethylamin mit CCMP ist exotherm, so dass die Umsetzung, aus Gründen der Verfahrenssicherheit, bevorzugt unter milderen Reaktionstemperaturen erfolgt.

Es wurde überraschend gefunden, dass bei zusätzlichem Einsatz eines Phasentransferkatalysators (PTC) die Reaktion unter milderen Bedingungen durchgeführt werden kann. Daher ist auch diese alternative Verfahrensvariante Gegenstand der Erfindung.

Das molare Verhältnis der anorganischen Base (bezogen auf OH⁻) zu dem eingesetztem CCMP der Formel (II) in dem erfindungsgemäßen Verfahren liegt bevorzugt im Bereich von 10:1 bis 0,1:1. Besonders bevorzugt liegt es im Bereich von 5:1 bis 0,5:1, ganz besonders bevorzugt im Bereich von 2:1 bis 1:1. Der Einsatz größerer Mengen anorganischer Base ist grundsätzlich möglich, jedoch in der Regel unwirtschaftlich. Die anorganische Base kann auch in katalytischen Mengen eingesetzt werden.

Im erfindungsgemäßen Verfahren wird 2,2-Difluorethylamin der Formel (I) bevorzugt im Überschuss eingesetzt. Das molare Verhältnis von CCMP der allgemeinen Formel (II) zum eingesetzten 2,2-Difluorethylamin liegt bevorzugt im Bereich von 1 : 1,5 bis 1 : 20, besonders bevorzugt im Bereich von 1 : 2 bis 1 : 10, ganz besonders bevorzugt von 1 : 2,5 bis 1 : 5.

Da die Edukte flüssige sind, kann das erfindungsgemäße Verfahren ohne ein zusätzliches Lösungsmittel für die Reaktion durchgeführt werden. Selbstverständlich kann auch in Anwesenheit eines Lösungsmittels gearbeitet werden.

Die erfindungsgemäße Reaktion kann in einem weiten Temperaturbereich (z.B. im Bereich von 1°C bis 100°C) durchgeführt werden. Vorzugsweise wird die Umsetzung in einem Temperaturbereich von 30°C bis 60°C durchgeführt.

Die Umsetzung wird bevorzugt unter Normaldruck, d.h. (950 - 1050 mbar absolut) durchgeführt. Das Verfahren kann prinzipiell aber auch bei erhöhtem Druck oder vermindertem Druck durchgeführt werden.

Die Reaktionsdauer der Reaktion ist kurz und liegt bevorzugt im Bereich von 0,5 bis 5 Stunden. Eine längere Reaktionsdauer ist möglich, jedoch in der Regel wirtschaftlich nicht sinnvoll.

Zur Aufarbeitung des Reaktionsgemisches werden die Überschüsse an eingesetztem 2,2-Difluorethylamin (DFEA) bevorzugt destillativ wiedergewonnen und können wiedereingesetzt werden.

Nach der Destillation von DFEA kann die Reaktionsmischung optional bevorzugt mit einem inerten Lösungsmittel - wie beispielsweise Toluol, Xylol, Butyronitril oder n-Butanol - und mit Wasser versetzt werden und das DFEA anschließend abgetrennt werden.

Nach optionaler pH-Wert-Einstellung der Lösung bevorzugt auf Werte von 5,5 - 6 wird N-[(6-Chlorpyridin-3-yl)methyl]-2,2-difluorethan-1-amin abgetrennt. Das 2,2-Difluorethylamin-Derivat der Formel (III) kann anschließend unter Normaldruck oder im Vakuum, vorzugsweise durch Destillation, isoliert werden.

Bevorzugt wird die anorganische Base in Substanz oder in Form einer wässrigen Lösung eingesetzt. In einer bevorzugten Ausführungsform wird daher das erfindungsgemäße Verfahren in Gegenwart von Wasser und zusätzlich in Gegenwart eines Phasentransferkatalysators (PTC) durchgeführt.

Phasentransferkatalysatoren (PTC) sind dem Fachmann grundsätzlich bekannt. Bevorzugt wird genau ein Phasentransferkatalysator eingesetzt. Es können aber auch zwei oder noch mehr verschiedene Phasentransferkatalysatoren eingesetzt werden. Besonders geeignete und bevorzugt eingesetzte Phasentransferkatalysatoren sind organische Ammonium- oder Phosphoniumsalze, insbesondere Tetraalkylammoniumsalze, Benzyltrialkylammoniumsalze, Tetraalkylphosphonium-salze, Benzyltrialkylphosphoniumsalze sowie Gemische davon.

Dabei werden bevorzugt organische Ammoniumsalze, insbesondere Tetraalkylammoniumsalze, Benzyltrialkylammoniumsalze verwendet. Solche Salze sind z.B. Tetra-n-butylammoniumchlorid oder - bromid, Tetra-n-butylammoniumhydrogensulfat, Tri-n-butylmethylammoniumchlorid oder -bromid, Tri-n-butylmethylammoniumhydrogensulfat, Benzyltriethylammoniumchlorid oder -bromid, Benzyltriethylammoniumhydrogensulfat, Trioktylmethylammoniumchlorid oder -bromid und Trioktylmethylammoniumhydrogensulfat.

Besonders bevorzugt wird das kommerziell erhältliche Tetra-n-butylammoniumchlorid oder -bromid sowie das kommerziell erhältliche Trioktylmethylammoniumchlorid verwendet.

Der Phasentransferkatalysator wird in katalytischen Mengen eingesetzt und kann vom Fachmann durch Routineexperimente ausgewählt und die geeignete Konzentration ermittelt werden.

Dennoch ist es von Vorteil, wenn die Menge des verwendeten Phasentransferkatalysators im Bereich von 0,01 bis 30 Mol-%, bezogen auf CCMP der Formel (II) liegt. Vorzugsweise liegt sie im Bereich von 0,05 bis 5, besonders bevorzugt im Bereich von 0,1 bis 3 Mol-%, bezogen auf das CCMP der Formel (II).

Durch Verwendung eines Phasentransferkatalysators kann die Umsetzung unter milderen Reaktionsbedingungen durchgeführt werden. Dies kann aus Gründen der Verfahrenssicherheit gefordert sein und hat auch den Vorteil, dass weniger Nebenkomponenten entstehen. Die Reaktionsmischung kann einfacher aufgearbeitet werden, was wiederum zu höheren Ausbeuten des gewünschten Zielprodukts führen kann.

Das Verwenden eines Phasentransferkatalysators ermöglicht, dass das Verfahren unter milderen Reaktionsbedingungen durchgeführt werden kann und damit wirtschaftlich vorteilhafter ist.

In einer anderen bevorzugten Ausführungsform wird das Verfahren in Abwesenheit eines Phasentransferkatalysators durchgeführt. Es ist überraschend, dass das Verfahren auch in Abwesenheit eines Phasentransferkatalysators durchgeführt werden kann, obwohl das Reaktionssystem zweiphasig ist.

### Beispiele:

Die vorliegende Erfindung wird anhand der nachfolgenden Beispiele näher erläutert, ohne die Erfindung dabei auf diese einzuschränken.

### A. Versuchsdurchführung

### Beispiel 7 (Versuch 7, erfindungs gemäß)

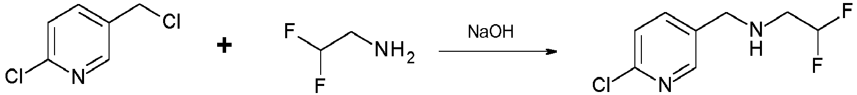

3684,7 g (45,0 mol) 2,2-Difluorethylamin (DFEA, GC-Gehalt: 99%) und 1931,1 g (15,45 mol; 32%) NaOH werden auf 55°C erhitzt. Zu dieser Mischung werden 496 g (CCMP, 3 mol, GC-Gehalt: 98%) einer Schmelze von CCMP innerhalb von 0,5 Stunden bei ca. 55°C zugetropft. Zunächst wird die Reaktionsmischung 2 Stunden bei 55 °C nachgerührt. Anschließend werden weitere 1983,9 g (CCMP, 12 mol, 98%) CCMP innerhalb von 2 Stunden zugetropft. Die Suspension wird 2 Stunden bei ca. 55 °C nachgerührt. Überschüssiges DFEA wird im Vakuum bei ca. 55 °C und zwischen 325 und 120 mbar abdestilliert und nach Zugabe von 1968 g Butanol und 1750 g Wasser wird die organische Phase abgetrennt. Man erhält 5420,8 g einer Lösung von N-[(6-Chlorpyridin-3-yl)methyl]-2,2-difluorethan-1-amin in n-Butanol mit einer GC-Reinheit von 55,7 Gew.-%. Nach GC-Methode mit externem Standard wird eine chemische Ausbeute von 97,40 % an N-[(6-Chlorpyridin-3-yl)methyl]-2,2-difluorethan-1-amin bezogen auf eingesetztes CCMP erhalten.

### Beispiel 6 (Versuch 6, erfindungsgemäß mit TBAB):

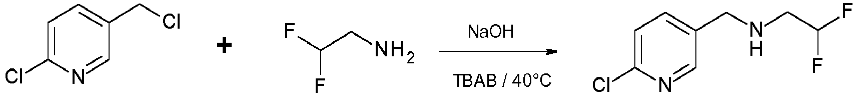

121,60 g (1,50 mol) 2,2-Difluorethylamin (DFEA, GC-Gehalt: 98%), 1,21 g Tetra-n-butylammoniumbromid (TBAB, 3,75 mmol) und 64,37 g (0,52 mol; 32%) NaOH werden auf 40°C erhitzt. Zu dieser Mischung werden 16,29 g (0,1 mol, GC-Gehalt: 99,5%, destilliertes CCMP) CCMP innerhalb von 0,5 Stunden bei ca. 40°C zugetropft. Zunächst wird die Reaktionsmischung 2 Stunden bei 40 °C nachgerührt. Anschließend werden weitere 65,16 g (0,4 mol, GC-Gehalt: 99,5%, destilliertes CCMP) CCMP innerhalb von 2 Stunden zugetropft. Die Suspension wird 2 Stunden bei ca. 40 °C nachgerührt. Überschüssiges DFEA wird im Vakuum bei 40 °C abdestilliert und nach Zugabe von 89,8 g Butyronitril und 75 g Wasser wird die organische Phase abgetrennt. Man erhält 212 g einer Lösung von N-[(6-Chlorpyridin-3-yl)methyl]-2,2-difluorethan-1-amin in Butyronitril mit einer GC-Reinheit von 44,9%. Nach GC-Methode mit externem Standard wird eine chemische Ausbeute von 92,12 % an N-[(6-Chlorpyridin-3-yl)methyl]-2,2-difluorethan-1-amin bezogen auf eingesetztes CCMP erhalten.

Nur Beispiel 6 wurde bei milderen Bedingungen von 40°C durchgeführt, wobei die Reaktion vor Reaktionsende abgebrochen wurde. Daher sind die erzielten Ausbeuten geringer als in den Beispielen 7 - 14, in denen ebenfalls NaOH als anorganische Base eingesetzt wurde.

### Beispiele 1 - 5 und 8 - 15 und 17:

Alle anderen Beispiele mit und ohne Verwendung von TBAB und der entsprechenden Base (Beispiele 1 - 5 und 8 - 15 und 17) wurden analog zu Versuch 7 durchgeführt, wobei in den Versuchen mit TBAB das anfängliche Reaktionsgemisch DFEA, die Base und TBAB enthielt und auf 55°C erhitzt wurde.

### Beispiel 16 (gemäß WO-A-2007/115644) unter Einsatz von Triethylamin als Base

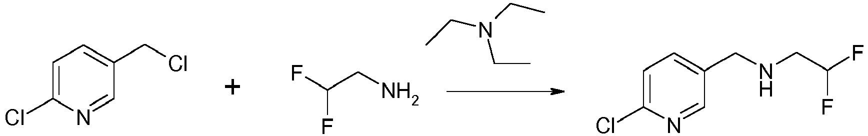

74,4 g (0,90 mol) 2,2-Difluorethylamin (Gehalt: 98%) und 48,5 g (0,48 mol) Triethylamin (Gehalt: 99%) werden auf 55°C erhitzt. Zu dieser Mischung werden 49,5 g (0,30 mol) CCMP (Gehalt: 98%) innerhalb von 2,5 Stunden bei dieser Temperatur zugetropft. Die gelbe Lösung läßt man 2 Stunden bei dieser Temperatur nachrühren und destilliert anschließend 74,0 g einer Mischung aus 2,2-Difluorethylamin und Triethylamin ab.

Nach GC-Methode mit externem Standard entspricht die Wiedergewinnung an 2,2-Difluorethylamin-Überschuss 0,50 mol (83%) und die Wiedergewinnung Triethylamin-Überschuss 0,11 mol (60%)

Der Rückstand wird mit 217 g Toluol und 55 g Wasser versetzt, auf 20°C abgekühlt und mit 20%-iger Salzsäure ein pH-Wert von 6 eingestellt. Die untere wäßrige Phase wird abgetrennt und das Lösungsmittel der organischen Phase destillativ entfernt.

Nach HPLC-Methode mit externem Standard wird eine chemische Ausbeute von 62% an N-[(6-Chlorpyridin-3-yl)methyl]-2,2-difluorethan-1-amin bezogen auf eingesetztes CCMP erhalten.

### Beispiel 17 unter Einsatz von Triethylamin als Base (nicht erfindungs gemäß)

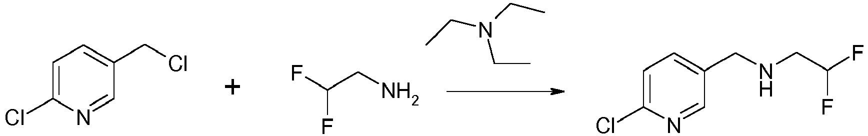

86,0 g (1,05 mol) 2,2-Difluorethylamin (DFEA, GC-Gehalt: 99%) und 36,85 g (0,36 mol; 99%) Triethylamin werden auf 55°C erhitzt. Zu dieser Mischung werden 11,57 g (CCMP, 0,07 mol, GC-Gehalt: 98%) einer Schmelze von CCMP innerhalb von 0,5 Stunden bei ca. 55°C zugetropft. Zunächst wird die Reaktionsmischung 2 Stunden bei 55 °C nachgerührt. Anschließend werden weitere 46,29 g (CCMP, 0,28 mol, 98%) CCMP innerhalb von 2 Stunden zugetropft. Die Suspension wird 2 Stunden bei ca. 55 °C nachgerührt. Überschüssiges DFEA wird im Vakuum bei ca. 55 °C und zwischen 325 und 120 mbar abdestilliert und nach Zugabe von Butanol und Wasser wird die organische Phase abgetrennt. Man erhält 211 g einer Lösung von N-[(6-Chlorpyridin-3-yl)methyl]-2,2-difluorethan-1-amin in n-Butanol mit einer GC-Reinheit von 27,94 Gew.-%. Nach GC-Methode mit externem Standard wird eine chemische Ausbeute von 81,87 % an N-[(6-Chlorpyridin-3-yl)methyl]-2,2-difluorethan-1-amin bezogen auf eingesetztes CCMP erhalten.

### B. Ergebnisse:

### (Versuche 1-5, 15, 16 und 17 nicht erfindungsgemäß, Versuche 6-14 erfindungsgemäß)

**Tabelle 1**

| **Versuch** | **Base** | **Phasentransfer katalysator [mol%]** | **Lösungsmittel** | **Extraktions mittel** | **Ausbeute [Std. GC-%]** |
|---|---|---|---|---|---|
| 1 | Hünigbase | ohne | n-PrCN | n-PrCN | 92,9 |
| 2 | ohne | ohne | 4 eq. DFEA | ohne | 88,7 |
| 3 | Na₂CO₃ | ohne | Wasser/DFEA | n-PrCN | 72,4 |
| 4 | KOH | 3 mol% TBAB | ohne | n-Butanol | 84,5 |
| 5 | Na₂CO₃ | 3 mol% TBAB | ohne | n-Butanol | 82,5 |
| 6^{40°C} | NaOH | 0,75 mol% TBAB | ohne | n-PrCN | 93,1 |
| 7 | NaOH | ohne | ohne | n-Butanol | 97,4 |
| 8 | NaOH | ohne | n-PrCN | n-PrCN | 98,4 |
| 9 | NaOH | ohne | Xylol | Xylol | 97,2 |
| 10 | NaOH | 2 mol% TBAB | ohne | n-Butanol | 96,7 |
| 11 | NaOH | 1 mol% TBAB | ohne | n-Butanol | 96,0 |
| 12 | NaOH | 0,5 mol% TBAB | ohne | n-Butanol | 95,2 |
| 13 | NaOH | 0,5 mol% TBAB | ohne | n-Butanol | 95,2 |
| 14 | NaOH | 0,75 mol% TBAB | ohne | n-PrCN | 98,8 |
| 15 | Na₂CO₃ | ohne | Wasser/DFEA | n-PrCN | 72,4 |

| | | | | | |
|---|---|---|---|---|---|
| TBAB: Tetra-n-butylammoniumbromid (Phasentransferkatalysator). Hünigbase: N,N-Diisopropylethylamin (Base aus WO-A-2014/001245). n-PrCN: Butyronitrile. KOH (85 Gew.-%) DFEA: 2,2-Difluorethylamin. eq.: Äquivalente. | | | | | |

Die Ergebnisse (Versuche 1 - 17) zeigen, dass
- die Reaktion mit anorganischen Basen mit guten Ausbeuten durchgeführt werden kann, wobei gleichzeitig alle bei Einsatz der Hünigbase verbundenen Nachteile (schlechte großtechnische Verfügbarkeit, aufwendige Aufbereitung und Wiedergewinnung etc.) vermieden werden können,
- die Reaktion mit NaOH mit deutlich besseren Ausbeuten durchgeführt werden kann als die Reaktion bei Einsatz von Triethylamin als Base (Beispiele 16 und 17 gemäß WO-A-2007/115644),
- bei Einsatz von NaOH als anorganischer Base deutlich höhere Ausbeuten erzielt werden konnten als bei Einsatz anderer anorganischer Basen (Na₂CO₃). Die Ausbeuten bei Einsatz von NaOH als anorganischer Base lagen zwischen 93,1% und 98,8% (Beispiele 6 - 14) während bei Einsatz von Na₂CO₃ oder KOH als anorganischer Base lediglich Ausbeuten im Bereich von 72,4% und 84,5% erzielt wurden (Beispiele 3, 4, 5 und 15).
- bei Einsatz von NaOH als anorganischer Base die höchsten Ausbeuten erzielt werden konnten (93,1% - 98,8%),
- die Umsetzung in Anwesenheit und in Abwesenheit eines Phasentransferkatalysators (TBAB) gelingt.

## Patentansprüche

1. Verfahren zur Herstellung von N-[(6-Chlorpyridin-3-yl)methyl]-2,2-difluorethan-1-amin der Formel (III) 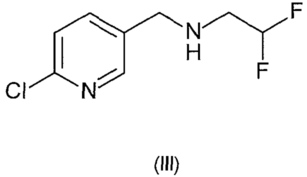 in dem 2,2-Difluorethylamin der Formel (I) 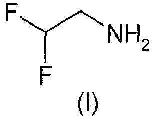 mit 2-Chlor-5-(chlormethyl)pyridin der Formel (II) 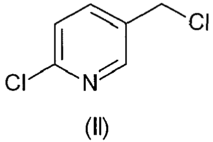 in Gegenwart einer anorganischen Base umgesetzt wird, wobei die anorganische Base

2. Verfahren nach Anspruch 1, bei dem die Umsetzung in Gegenwart eines Phasentransferkatalysators durchgeführt wird.

3. Verfahren nach Anspruch 2, bei dem der Phasentransferkatalysator ein organisches Ammonium- oder Phosphoniumsalz ist.

4. Verfahren nach Anspruch 1, bei dem die Umsetzung in Abwesenheit eines Phasentransferkatalysators durchgeführt wird.

5. Verfahren nach einem der Ansprüche 1 bis 4, bei dem das unverbrauchte 2,2-Difluorethylamin der Formel (I) nach der Umsetzung abdestilliert wird und anschließend dem Verfahren als Edukt wieder zugeführt wird.

## Claims

1. Process for preparing N-[(6-chloropyridin-3-yl)methyl]-2,2-difluoroethan-1-amine of formula (III) 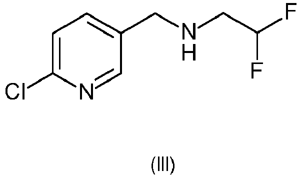 wherein 2,2-difluoroethylamine of formula (I) 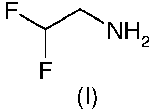 is reacted with 2-chloro-5-(chloromethyl)pyridine of formula (II) 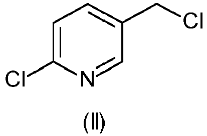 in the presence of an inorganic base, wherein the inorganic base is NaOH.

2. Process according to Claim 1, wherein the reaction is carried out in the presence of a phase-transfer catalyst.

3. Process according to Claim 2, wherein the phase-transfer catalyst is an organic ammonium or phosphonium salt.

4. Process according to Claim 1, wherein the reaction is carried out in the absence of a phase-transfer catalyst.

5. Process according to any of Claims 1 to 4, wherein the unconsumed 2,2-difluoroethylamine of formula (I) is distilled off following the reaction and subsequently returned to the process as reactant.

## Revendications

1. Procédé de fabrication de N-[(6-chloropyridin-3-yl)méthyl]-2,2-difluoroéthan-1-amine de formule (III) 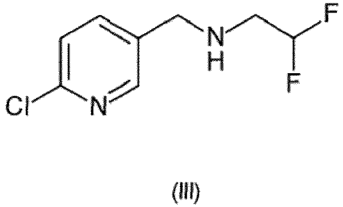 selon lequel de la 2,2-difluoroéthylamine de formule (I) 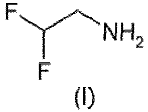 est mise en réaction avec de la 2-chloro-5-(chlorométhyl)pyridine de formule (II) 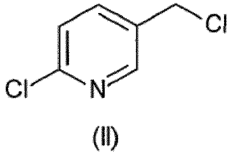 en présence d'une base inorganique, la base inorganique étant NaOH.

2. Procédé selon la revendication 1, selon lequel la réaction est réalisée en présence d'un catalyseur de transfert de phase.

3. Procédé selon la revendication 2, selon lequel le catalyseur de transfert de phase est un sel d'ammonium ou de phosphonium organique.

4. Procédé selon la revendication 1, selon lequel la réaction est réalisée en l'absence d'un catalyseur de transfert de phase.

5. Procédé selon l'une quelconque des revendications 1 à 4, selon lequel la 2,2-difluoroéthylamine de formule (I) non utilisée est éliminée par distillation après la réaction, puis réintroduite dans le procédé en tant que réactif.
